# EUROPEAN PATENT SPECIFICATION

(11) **EP 3 917 611 B1**
(45) Date of publication and mention of the grant of the patent: **15.10.2025**
(21) Application number: 20749282.8
(22) Date of filing: 31.01.2020
(51) Int. Cl.: A61N 1/36, A61N 1/05, A61N 1/372, A61B 5/11, A61B 5/00, A61F 2/12, A61F 2/68, A61N 1/375, A61B 5/0205

(54) **BIONIC BREAST**
BIONISCHE BRUST
SEIN BIONIQUE

(30) Priority: 31.01.2019 US 201962799568 P
(43) Date of publication of application: 08.12.2021
(73) Proprietor: The University of Chicago, Chicago, IL 60637 (US)
(72) Inventor: LINDAU, Stacy, Tessler, Chicago, IL 60637 (US); BENSMAIA, Sliman, Chicago, IL 60637 (US); FELDMETH, Gillian, Chicago, IL 60637 (US); LONG, Katie, Chicago, IL 60637 (US); WANG, Sihong, Chicago, IL 60637 (US)
(74) Representative: Marks & Clerk LLP
(86) International application number: PCT/US2020/016255
(87) International publication number: WO 2020/160498

(56) References cited:
- WO-A1-2017/044904
- WO-A1-2017/214638
- WO-A1-2018/071494
- US-A1- 2009 012 372
- US-A1- 2010 241 191
- US-A1- 2015 148 878
- US-B2- 7 239 918
- US-B2- 7 565 198
- US-B2- 8 938 299

## Description

### CROSS-REFERENCE TO RELATED APPLICATIONS

This application claims priority to U.S. Provisional Application No. 62/799,568, filed January 31, 2019.

### BACKGROUND

Breast reconstruction may be performed to restore breast aesthetics. Reconstruction may occur following mastectomy to treat or prevent breast cancer, following injury, or for some other reason. However, both mastectomy and breast reconstruction (and/or other surgical or radiation procedures performed on the breast or surrounding tissues) can result in diminishment or complete loss of sensory function of the breast. This loss or diminishment of sensory function can have a variety of negative psychological, physiological, social, and/or relational effects, including sexual dysfunction and/or diminishment or loss of sexual function. Such diminishment or loss of sexual function or sexual dysfunction due to medical alterations to the breast can occur in either or both of the person whose breast is treated and the intimate or sexual partner of this person. Therefore, there is a need to improve sensory function of the breast in post-operative and other individuals who experience sensory loss in the breast. WO2017/214638A1 relates to a system for wireless recording and stimulating of bioelectric events. US2015/148878A1 relates to systems and methods of enhancing electrical activation of nervous tissue. US2010/241191A1 relates to methods and apparatus for assisting deglutition. WO2018/071494A1 relates to systems and methods for controlling levels of perceived intensity of a sensory stimulus. US2009/012372A1 relates to external sensing for implant rupture.

### SUMMARY

The present invention is defined by the appended claims and provides a system as defined in claim 1.

Methods disclosed hereinafter are not claimed as such but are exemplary nature and provided for better understanding the invention.

An aspect of the present disclosure relates to a system including: (i) a sensor, wherein the sensor is configured for subcutaneous placement and wherein the sensor is configured to detect at least one of a pressure, a strain, a vibration, or a temperature; (ii) a stimulator, wherein the stimulator is configured to stimulate at least one nerve; and (iii) a controller, wherein the controller is operably coupled to the sensor and the stimulator. The controller is configured to: (a) operate the sensor to detect at least one of a pressure, a strain, a vibration, or a temperature; and (b) based on the detected at least one of a pressure, a strain, a vibration, or a temperature, operate the stimulator to provide a stimulus to the at least one nerve

Also disclosed herein is a system including: (i) a breast implant; (ii) a sensor, wherein the sensor is disposed on or within the breast implant proximate to a surface of the breast implant, wherein the breast implant is configured for subcutaneous placement, and wherein the sensor is configured to detect at least one of a pressure, a strain, a vibration, or a temperature; (iii) a stimulator, wherein the stimulator is configured to stimulate at least one nerve; and (iv) a controller, wherein the controller is operably coupled to the sensor and the stimulator. The controller is configured to: (a) operate the sensor to detect at least one of a pressure, a strain, a vibration, or a temperature; and (b) based on the detected at least one of a pressure, a strain, a vibration, or a temperature, operate the stimulator to provide a stimulus to the at least one nerve.

Also disclosed herein is an exemplary method including: (i) detecting electrical activity in a set of multiple nerves located in a chest of a person; (ii) while detecting the electrical activity, providing stimulus to multiple locations of a breast of the person; (iii) based on the detected electrical activity, identifying, from the set of multiple nerves, a set of relevant nerves; and (iv) implanting, into the person, a system. The system includes: (a) a sensor, wherein the sensor is configured to detect at least one of a pressure, a strain, a vibration, or a temperature; (b) a stimulator, wherein the stimulator is configured to stimulate at least two nerves, and wherein the stimulator comprises a plurality of electrodes; and (c) a controller. The controller is operably coupled to the sensor and the stimulator and is configured to: operate the sensor to detect at least one of a pressure, a strain, a vibration, or a temperature; and, based on the detected at least one of a pressure, a strain, a vibration, or a temperature, operate the stimulator to provide a stimulus to at least one of the at least two nerves. Implanting the system includes coupling each electrode of the plurality of electrodes to a respective nerve of the set of relevant nerves.

Also disclosed herein is an exemplary method for restoring breast sensation to a person, the method including: (i) detecting, using a sensor implanted in a breast of a person, at least one of a pressure, a strain, a vibration, or a temperature; and (ii) based on the detected at least one of a pressure, a strain, a vibration, or a temperature, operating a stimulator to provide a stimulus to one or more intercostal nerves of the person.

These as well as other aspects, advantages, and alternatives will become apparent to those of ordinary skill in the art by reading the following detailed description with reference where appropriate to the accompanying drawings. Further, it should be understood that the description provided in this summary section and elsewhere in this document is intended to illustrate the claimed subject matter by way of example and not by way of limitation.

### BRIEF DESCRIPTION OF THE DRAWINGS

Figure 1 depicts elements of an example system that has been implanted into a person.
Figure 2A depicts elements of an example system that has been implanted into a person.
Figure 2B depicts elements of an example system that has been implanted into a person.
Figure 3 depicts elements of an example system that may be used to implement the methods described herein.
Figure 4 is a flowchart of an example method.
Figure 5 is a flowchart of an example method.

### DETAILED DESCRIPTION

In the following detailed description, reference is made to the accompanying figures, which form a part hereof. In the figures, similar symbols typically identify similar components, unless context dictates otherwise. The illustrative embodiments described in the detailed description, figures, and claims are not meant to be limiting. Other embodiments may be utilized, and other changes may be made, without departing from the scope of the present invention as defined by the appended claims. It will be readily understood that the aspects of the present disclosure, as generally described herein, and illustrated in the figures, can be arranged, substituted, combined, separated, and designed in a wide variety of different configurations, all of which are explicitly contemplated herein.

This application makes reference to the contents of Xu et al., "Highly stretchable polymer semiconductor films through the nanoconfinenment effect," and Wang et al., "Skin electronics from scalable fabrication of an intrinsically stretchable transistor array", Nature, Vol. 555, March 1, 2018.

### I. Overview

Mastectomy, breast reconstruction, or other surgical or radiation interventions to the breast and/or surrounding tissues (e.g., the intercostal nerves that innervate the breast) or certain anatomic conditions or injuries can result in diminished or lost sensation of the breast or other reductions in the various natural functions of the breast. While the surgical techniques or approaches employed to obtain a particular surgical, radiation, or other treatment goal can be adapted to spare the relevant nervous tissue, such adaptation may not be available in every case. Further, such adaptations may not always be successful, resulting in diminished or lost sensory or other aspects of breast function. Such surgical or other therapeutic interventions can include mastectomy, radiation, or other procedures to remove or otherwise diminish cancerous tissue in the breast or surrounding tissue, gender reassignment procedures, reconstruction following injury, elective cosmetic procedures, or other procedures that may result in loss or diminishment of breast function.

To restore breast function lost due to surgical intervention or other medical treatment or trauma, systems (e.g., worn or implanted devices, materials, monitors, information systems, or some combination thereof) may be provided. In particular, breast sensory and other functions may be restored in an individual by implanting or otherwise placing one or more sensors in or on the breast of the individual to detect physical variables such as movement, vibration, pressure (e.g., deep, superficial, fine, and/or gross pressure), wetness, suction, air movement, temperature, strain, deformation, accelerations, or other physical variables related to a lost sensation (e.g., proprioception, touch, texture, erection of the nipple (e.g., in response to physiological arousal), tingling, fullness, heat, or other sensations). These detected physical variables can then be used to determine a stimulus that is provided to the central (and/or peripheral) nervous system of the individual (e.g., via electrical stimulation of one or more peripheral nerves) such that a percept physiologically relevant to the detected physical variable(s) is experienced by the individual.

A contemplated device can include one or more sensors, one or more electrodes and/or other elements of a stimulator, and a controller configured to use the sensor(s) to detect information and to generate, via the stimulator, stimulation to evoke a perception related to the detected information (including but not limited to a touch, a vibration, a texture, a pressure, a motion, a deformation, heat, cold, or some combination thereof). Such a device may include other components to facilitate the operation of the device. For example, the device may include one or more batteries, a coil and/or other element(s) for wirelessly receiving power (e.g., to recharge a battery of an implanted device by receiving power from outside a body), or other components to power the device. The device may include wireless receivers and/or transmitters to facilitate programming, controlling, and/or monitoring the device or information or other signals generated by the device and/or its biological interfaces. Wireless components are further contemplated to enable different components of the device to functionally interact without being physically connect, such as by wiring. The device may include reed switches, magnetometers, and/or other elements for receiving inputs to control the device, e.g., by passing a magnetic control pendant over the device.

The device may be fully or partially implantable. In some examples, the device can be wholly implanted, with the sensor, controller, stimulator, and other elements implanted at respective locations within an individual's body. For example, the sensor can be implanted within or near the skin of the breast or at some other location within or near the breast, while electrodes or other elements of the stimulator can be located near, within, and/or enclosing one or more nerves. In some examples, the device can include implanted elements and external elements. For example, the stimulator and electrodes or other elements thereof can be implanted while the sensor is worn outside of the body (e.g., adhered to the surface of the breast), and signals can be transmitted from the sensor to the stimulator. In another example, the device can include one or more transcutaneous elements, e.g., an electrode or lead that passes through the skin to deliver stimulation to a nerve from a stimulator location outside the body.

The sensor(s) of the device may include a variety of elements configured to detect stress, strain, vibration, pressure, deformation, motion (e.g., relative or absolute velocity and/or acceleration), orientation (e.g., relative to gravity), relative location (e.g., relative location between elements of the implanted device and/or between elements of the device and portions of a user's anatomy), temperature, heart rate, respiratory rate, blood pressure, vasodilation, or some other physical variable(s) related to the breast, to sensations that would be experienced via an intact breast under similar conditions, and/or to the behavioral, social, physiological, and/or emotional context of such sensations.

The sensor(s) can include strain sensors, thermistors, flexible resistive elements, optical fibers, pressure transducers, accelerometers, gyroscopes, light emitters and/or light sensors, or other elements to detect one or more physical variables. In some examples, the sensor(s) can be configured to have a compliance that corresponds to the compliance of breast tissue and/or of skin tissue (e.g., to have a compliance within 30% of such tissue, or within 15% of such tissue, or a compliance that is indistinguishable from such tissue by the user). For example, the sensor(s) can be manufactured from flexible materials, can be formed from a mesh of material, can have a thickness or other dimension less than a maximum dimension, or can be configured in some other manner to reduce the compliance (i.e., to increase the flexibility) of the sensor(s) and/or to match the compliance of the sensor(s) to tissues in and/or near which the sensor is implanted. Such compliance reduction/matching can be accomplished to improve the tactile cosmesis of the sensor(s) or to otherwise reduce or minimize the intrusiveness of the sensor(s) when located in or on the breast or related tissue. The device can include multiple sensors located (e.g., implanted) and can be configured to detect physical variables related to different portions of the breast, e.g., from a first location proximate to the nipple/areola and from a second, different location.

The sensor(s) can be implanted subcutaneously and/or subdermally in a variety of locations, and fixed in place via a variety of methods or means. The location of the sensor may be specified to allow the sensor to detect pressures, strains, temperatures, vibrations, accelerations, deformations, or other physical variables related to touch or other percepts of the skin and/or bulk of the breast. Accordingly, the sensor may be implanted in an internal surface of the skin, e.g., some sub-epidermal surface or location of the skin. The surface can be a sub-dermal surface of the skin, or can be a surface created surgically, e.g., a surface within an intra-dermal space that has been formed by a surgeon. Alternatively, the sensor may be emplaced on an internal or external surface of muscle, or on or within fat or other tissues of the breast (e.g., to account for specifics of a particular person's anatomy and/or other surgical considerations). It is also contemplated that multiple sensors can be placed in different locations in or on the breast as described herein.

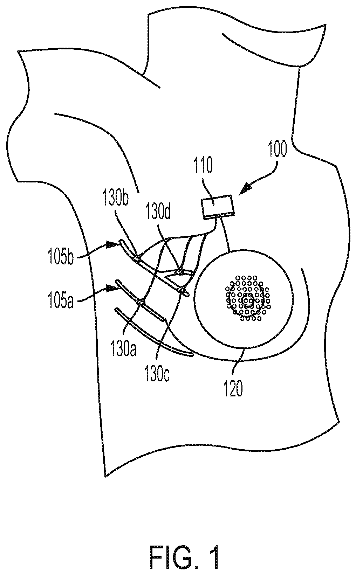

Figure 1 illustrates an example implantable system 100. The system 100 includes a controller 110 operably coupled (e.g., via respective leads or cables) to a sensor 120 (e.g., a flexible sensor) disposed beneath skin of a breast and electrodes disposed within respective electrode housings 130a-d so as to maintain the electrodes in proximity to respective nerves. As shown in Figure 1, the nerves can include roots or other portions of intercostal nerves 105a, 105b or other nerves in the chest (or elsewhere) of a person. The nerves may be selected according to a clinician's perspective as to which nerves are likely to innervate the skin of the nipple, areola, or other relevant areas of skin of a patient. Additionally or alternatively, the electrical activity within a set of nerves can be recorded, prior to reconstructive surgery or other possibly nerve-damaging procedures, while mechanical or other stimulation is provided to the nipple, areola, or other relevant areas of skin. The recorded electrical activity can then be used to identify which nerves to implant with electrodes. As shown, the roots of multiple different nerves (e.g., intercostal nerves 105a, 105b that can include the second, third, fourth, fifth, and/or sixth intercostal nerves) may be implanted with the electrodes located in electrode housings 130a and 130b, respectively. Additionally or alternatively, one or more roots of an intercostal nerve may be implanted with electrodes disposed in electrode housings 130c and 130d. It is further contemplated that multiple electrodes may be implanted in the same nerve. It is also contemplated that a single electrode may be in functional communication with multiple nerves.

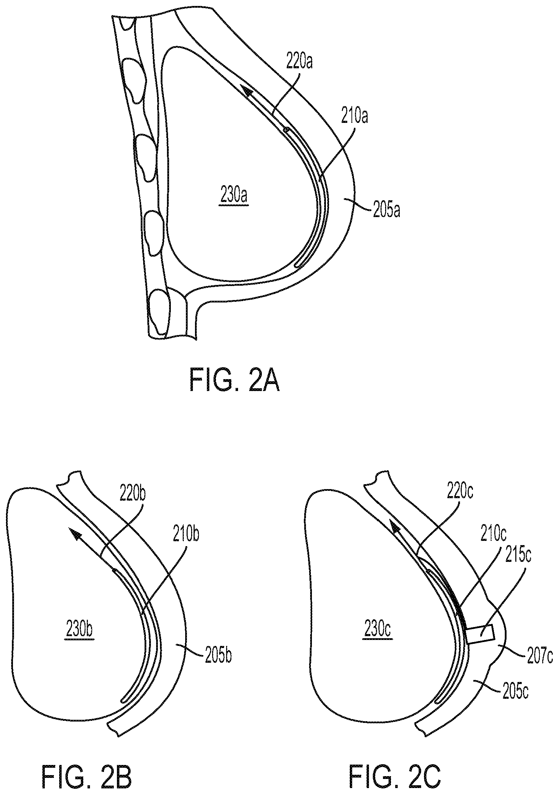

Figure 2A illustrates an example sensor 210a, with attached cabling 220a (e.g., that may lead to a controller or other component(s) of an implantable system as described herein) that has been surgically implanted within a breast. The sensor 210a has been secured to skin 205a of the breast (e.g., to an internal, sub-dermal surface of the skin) so as to facilitate the functionality described herein. A reconstructive breast implant 230a (e.g., a saline implant, a silicone implant, etc.) has also been surgically implanted within the breast.

To facilitate detection of physical variables (e.g., pressure, strain, motion, temperature, etc.) that are relevant to percepts related to the skin and/or bulk of the breast, the sensor(s) may be flexible sensors. Such flexibility in the sensor(s) may also provide benefits with respect to the visual and tactile cosmesis of the breast, by having the compliance of the sensor approximate the compliance of the tissues of the skin and/or breast (e.g., a compliance within 30% of the compliance of the skin and/or breast). Such a flexible sensor can include a number of non-flexible elements (e.g., discrete transistors, integrated circuits, sensor elements) embedded in a flexible substrate such that the sensor is, overall, flexible. Additionally or alternatively, such a flexible sensor can include flexible transistors, flexible capacitors (e.g., an array of flexible capacitive sensor elements that can be used to detect pressure, strain, vibration, or other physical variables), or other flexible electronic elements. For example, a flexible capacitor can be formed by providing a flexible dielectric material between two plates or otherwise-shaped portion of a flexible conductive material. Such a flexible capacitor can be made sensitive to pressure, strain, or other physical parameters by, e.g., providing a compressible, stretchable, or otherwise deformable dielectric and/or spacer material between the plates of the flexible capacitor. Such flexible capacitors or other flexible elements can be formed from or otherwise integrated into a flexible substrate material, e.g., a flexible conductive or semiconductive material.

The sensor can include an array of sensor elements (e.g., flexible capacitor sensor elements) having a spacing or resolution determined relative to the resolution of natural skin in a particular application. For example, the sensor may include an array of sensors with a resolution of one sensor per square millimeter or less so as not to exceed the natural touch sensor resolution of skin of the nipple areola complex.

The sensor can include tabs or other features to facilitate surgically fixing the sensor to anatomy of interest (e.g., to a sub-dermal surface or some other internal surface of skin). A flexible or otherwise configured sensor may also include a plurality of holes or other features formed therein to facilitate through-growth of tissue, securing the sensor to adjacent tissues. Such through-growth may allow resorbable sutures, clips, or other resorbable materials to be used to initially fix the sensor to adjacent tissues and later to be resorbed after the through-growth has occurred. This can have the benefit of avoiding the potentially negative tactile or other effects of sutures, clips, or other securing elements long after implantation of the sensor has occurred. The use of suture, clips, or other surgical fixing means that are not resorbable may result in negative effects on the tactile perception of the reconstructed breast, due to the long-term presence of such rigid, non-resorbable elements in the reconstructed breast. Thus, the presence of holes or other features to permit through-growth of tissue to fix the sensor in the breast long-term can be beneficial by allowing the use of resorbable sutures, clips, or other fixing means.

Many individuals who receive an implanted system as described herein may also receive a breast implant (e.g., a saline breast implant, a silicone breast implant) as part of breast reconstruction. Accordingly, it may be beneficial in some circumstances to incorporate one or more elements of a system as described herein (e.g., a flexible or otherwise configured sensor, a controller, a battery, portions of a stimulator, etc.) as part of such a breast implant. This can be done, e.g., to minimize the number of foreign bodies (breast implants, sensory stimulator systems, etc.) that are present in the body and/or to reduce the total amount of surface area, within the body, that is in contact with such foreign bodies.

For example, a sensor (flexible or otherwise) may be disposed on or near a surface of a breast implant such that, when the surface of the breast implant is fixed proximate to a skin surface (e.g., surgically sutured or otherwise fixed to a sub-dermal surface or some other internal surface of skin), the sensor can be operated to detect pressures, strains, vibrations, temperatures, or other physical variables relevant to functionally restoring sensation to the overlying skin. The breast implant and/or sensor disposed therein or thereon may include tabs or other features to facilitate fixing the sensor proximate to tissue of interest (e.g., to a sub-dermal or other internal surface of skin or of fascia or some other tissue). The breast implant and/or sensor disposed therein or thereon may additionally or alternatively include one or more features (e.g., tabs) into which are formed a plurality of holes or other features to facilitate through-growth of tissue to stabilize the sensor relative to the tissue.

Figure 2B illustrates an example sensor 210b, with attached cabling 220b (e.g., that may lead to a controller or other component(s) of an implantable system as described herein) that has been surgically implanted within a breast. The sensor 210b is disposed within and proximate to a surface of a breast implant 230b that has also been surgically implanted within the breast. The surface of the breast implant 230b is secured to skin 205b of the breast (e.g., to an internal, sub-dermal surface of the skin) so as to facilitate the functionality described herein. Alternatively, depending on the surgical considerations involved, the breast implant 230b and/or sensor 210b may be secured to a surface of muscle or to some other non-skin tissue (e.g., fat, fascia) of the breast.

As shown above, a sensor of a system as described herein (e.g., a flexible sensor) may include a substantially flat element that is configured to be placed beneath and substantially parallel to the external surface of the skin. Additionally or alternatively, an implantable sensor can be configured for implantation within a natural or reconstructed nipple so as to facilitate the restoration of functional sensation of stimuli to the nipple. Such a sensor can be flexible. Such a sensor can be composed of a ring, rod, strip, or other shape of material. Such a sensor can be continuous with or otherwise coupled to a substantially flat sensor element as described above (e.g., 120, 210a, 210b) or can be separate therefrom. Figure 2C illustrates an example flat sensor 210c and intra-nipple sensor 215c, with attached cabling 220c (e.g., that may lead to a controller or other component(s) of an implantable system as described herein) that has been surgically implanted within a breast. The flat sensor 210c has been secured to skin 205c of the breast (e.g., to an internal, sub-dermal surface of the skin) and the intra-nipple sensor 215c has been secured into tissue of a preexisting or reconstructed nipple 207c so as to facilitate the functionality described herein. A reconstructive breast implant 230c (e.g., a saline implant, a silicone implant) has also been surgically implanted within the breast.

A device as described herein can provide stimulus to one or more nerves (e.g., remnant nerves) electrically, optically, or according to some other mechanism. For example, a stimulator of the implanted device can include one or more electrodes configured to be implanted near and/or within a nerve and/or a fascicle thereof such that electrical currents and/or voltages can be provided, via the electrode(s), to stimulate the nerve and/or a fascicle thereof. The electrode(s) can be disposed on or within a housing to facilitate coupling the electrode(s) to a nerve and/or to stabilize the interface between the electrode(s) and the nerve. For example, the device can include a housing (e.g., a housing composed of a polymer material, a metal, a mesh, or some other material) configured to be implanted near, on, or around a nerve and/or fascicle. Such a housing can include tabs, flaps, holes, cuffs, rings or other features to facilitate suturing or otherwise securing or integrating the housing to tissue (e.g., to fascia associated with a nerve or to a nerve sheath).

The electrode(s) can include penetrating electrodes (e.g., intrafascicular electrodes), surface electrodes, enclosing electrodes (e.g., cuff electrodes), transverse intrafascicular multichannel electrodes, longitudinal intrafascicular multichannel electrodes, monopolar electrodes, multipolar electrodes (e.g., bipolar, tripolar), or some other variety of electrodes or combination of electrodes. The electrodes can be composed of metallic materials (e.g., gold, platinum, silver, platinum-iridium, tungsten), metallic oxides, polymeric materials (e.g., PEDOT), or other materials to facilitate electrical conduction, ionic conduction, capacitive coupling, mechanical stability, biocompatibility, or other considerations. The electrodes can include surface layers (e.g., a layer of silver/silver-chloride, a protective and/or biocompatible layer), surface textures or treatments (e.g., to increase the effective surface area of the electrodes), or other structures.

In some examples, the housing can wholly or partially enclose the nerve, (e.g., by having an elongated helical shape), by having an enclosing shape with a cut or cutout (e.g., a "C" shape), or some other shape to facilitate wholly or partially enclosing or integrating with a nerve. In such examples, the electrode(s) disposed on or within the housing can be disposed on an inner surface of the housing to facilitate electrical contact with the nerve. In some examples, the location of the electrodes can be specified such that each electrode preferentially provides stimulation to a respective portion of one or more fascicles within a nerve in order to facilitate finely graded control of the stimulation provided to the nerve. In some examples, the housing can have a shape and/or compliance to flatten the nerve, facilitating such electrode-selective stimulation.

The implanted device can include multiple housings (with associated respective electrode(s)) or other multiple elements to facilitate providing stimulation to more than one nerve and/or to more than one portion of a nerve (e.g., to different portions of an intercostal nerve). For example, the device can include two, three, or more housings (with respective one or more electrodes each) to facilitate stimulation of two, three, or more different nerves or nerve branches (e.g., two or three of the third, fourth, and fifth intercostal nerves, of the lateral branches thereof, and/or of some other intercostal nerves or other nerves of interest). Such multiple nerve stimulation can be provided in order to provide functional redundancy (e.g., in the event that a nerve is damaged during surgery or otherwise), to allow for the 'best' nerve of a set to be selected for stimulation during intra-operative fitting or other procedures to place the device, to allow percepts to be delivered by simultaneous stimulation of multiple nerves, or to provide some other benefit. The multiple housings or other elements configured to be coupled to respective different nerves can be electrically and mechanically coupled to a controller via respective different leads or via a common lead, e.g., to facilitate implantation.

Multiple nerves may be coupled to electrodes (or to sets of electrodes, e.g., a linear array of electrodes within a flat interface nerve electrode) to facilitate restoration of functional sensation in the nipple, areola, or other areas of the breast or body. This can include emplacing the electrodes on nerves of the chest, e.g., on one or more of the second, third, fourth, fifth, sixth, or other intercostal nerves and/or branches thereof. Electrodes may be emplaced on the root, lateral branch, or other branches of the intercostal or other nerves. Each nerve (e.g., root, branch, etc.) includes a plurality of nerve fibers (or axons), each of which innervates a respective (potentially overlapping) portion of the nipple, areola, or other skin of the breast. Thus, a relevant set of nerves may be selected for implantation such that the selected nerves may be stimulated to functionally restore sensation in the nipple, areola, or other portions of skin of interest. The relevant set of nerves may be selected according to which of the nerves naturally provided innervation to the nipple, areola, or other portions of skin of interest prior to resection, radiation therapy, mastectomy, reconstruction, or other medical interventions which may damage the connection between those nerves and the skin.

This selection may be performed based on a mapping process between stimuli provided to the nipple, areola, or other portions of skin of interest and electrical activity recorded from a set of candidate nerves (e.g., roots and/or branches of intercostal or other nerves of the chest that are accessible for stimulation and that are likely to contain nerves that provide sensory innervation to the nipple, areola, or other portions of skin of interest). Such a mapping may be identified prior to resection, radiation therapy, mastectomy, reconstruction, or other medical interventions which may damage the connection between those nerves and the skin. For example, electrical activity in a set of candidate nerves (e.g., one or more of the second, third, fourth, fifth, or sixth intercostal nerves and/or one or more branches thereof) may be detected intraoperatively during a tumor resection and/or mastectomy operation or during some other surgery. Mapping may also be performed preoperatively or postoperatively. This can include emplacing electrodes (e.g., contact electrodes, penetrating electrodes, one or more arrays of electrodes, or some other type or combination of types of electrodes) on the candidate nerves and detecting and/or recording electrical activity detected therefrom (e.g., local field potentials) while stimulus is provided to the breast.

Providing stimulus to the breast can include a surgeon or other healthcare provider manually providing the stimulus. Additionally or alternatively, the stimulus can be provided by an electrical stimulator, a mechanical vibrator or other mechanical actuator, a fan, or some other means to provide the stimulus. The stimulus can be provided to a specified set of locations, e.g., to a set of locations specified pre-operatively. The set of locations can be determined based on feedback from the person regarding the types and locations of sexual or other stimulation that are most valuable.

Based on the detected electrical activity, a set of relevant nerves (candidates for later stimulus by an implanted stimulator implant) can be identified from the set of multiple nerves from which the electrical activity was detected. This can be performed by a surgeon or other healthcare provider, e.g., based on correspondences in timing of the stimulus and a visually or acoustically-presented indication of the detected electrical activity in one or more of the multiple nerves. Additionally or alternatively, a computer can be used to identify the set of relevant nerves based on recordings of the detected electrical activity and one or more signals related to the provision of the provided stimulus, e.g., a signal related to the pressing of a button by a surgeon when the surgeon is providing stimulus to the breast.

The identified set of relevant nerves can then be coupled to respective electrodes of an implantable stimulator system as described elsewhere herein. Such an implantation may occur during the same surgical procedure during which the original identifying electrical activity was recorded. Alternatively, electrodes of a stimulator implant may be coupled to the identified nerves curing a subsequent surgery, e.g., during reconstructive surgery that follows a mastectomy other prior surgical procedure and that may also include the implantation of a breast implant or the performance of other reconstructive procedures that may result in damage to the connections between the identified relevant nerves and the skin of the breast. Where more nerves are present in the identified set of relevant nerves than can be coupled to respective electrodes of the implanted system (e.g., due to the system having fewer electrodes and/or electrode housings than there are nerves in the set of relevant nerves), a subset of the set of relevant nerves can be coupled to respective electrodes. Such a subset can be selected based on the strength of response of the nerves to the stimulus, based on the user's preferences, or based on some other consideration.

The device can operate in a single mode (e.g., translating physical variables detected using the sensor(s) into stimulation waveform(s) to provide corresponding percepts according to a single mapping or other algorithm (e.g., between detected pressure levels and an amplitude, a frequency, a pulse waveform, or some other property of a corresponding stimulus waveform)). Alternatively, the device can operate in two or more different modes. For example, the device can generally operate in a mode to translate detected physical variables according to a first mapping (or other algorithm) to provide touch or other percepts related to the breast (e.g., to provide a percept related to the feeling of a hug on the breast). However, the device can in certain circumstances operate in alternative modes (e.g., in a sexual mode) to translate detected physical variables according to a second mapping (or other algorithm) to provide touch, pressure, texture, or other percepts related to the breast (e.g., to provide a percept related to the feeling of a sexual touch on the breast).

Transition between such modes can occur as the result of an express command from a user (e.g., by passing a magnetic control pendant over an implanted device, by operating an app on a cellphone that is in wireless communication with the device) or based on the device detecting that a mode change is indicated (e.g., by operating the sensor(s) to detect that sexual activity is likely occurring). The transition can be sudden, or gradual over a period of seconds or minutes (e.g., by providing, during a transition period, stimulation that is somewhere between the stimulation for the first mode and the second mode). The device can operate along a continuum between modes or parameter settings, according to user input (e.g., a user setting a knob or slider of a user interface between two extremes). For example, a stimulation amplitude, frequency, or other stimulation parameter can be set, by a user operating a user interface, between a minimum value and a maximum value. Alternatively, no transition occurs.

The stimulation waveforms, nerves and/or fascicles thereof to be stimulated, the mappings between detected physical variables and parameters of the provided stimulus, or other parameters of operation of the device can be programmed following implantation, fitting, and/or donning of the device. These parameters can be selected from an enumerated set of parameter settings known to correspond to likely mappings between detected physical variables and stimulation likely to provide appropriate percepts. These mappings can be informed by the known response properties of nerve fibers innervating the breast (i.e., biomimetic mappings). Additionally or alternatively, the parameters may be set individually. The device programming may occur in a health care setting (e.g. physician's office, hospital) and/or may occur in the home (e.g., by a home nurse or device professional or a user programming the device using an app on a cellphone that is in wireless communication with the device).

Some of the parameters of operation of the device can be set based on pre-implantation or pre-placement (e.g., pre-mastectomy, pre-reconstruction, pre-gender reassignment) information on the sensory and other function of the intact breast, e.g., by performing nerve blocks, followed by erotic or other stimulation, in order to map which nerves (e.g., intercostal nerves) or portions of nerves provide which types of innervation to relevant portions of the breast. In some examples, neurograms or other recordings of nervous activity in the intact breast during erotic or other stimulation can be used to determine appropriate stimulation parameters and/or mapping(s) between such stimulation parameters and detected physical variables of the breast. In instances where only one breast is affected, parameters of operation for the affected breast can be set by measurements of sensory function of the remaining, intact breast.

In some examples, a device as described herein is configured and operated to mimic and recapitulate the 'natural' function, and associated percepts, of the breast. Alternatively, such devices can be configured and operated to "replicate" the sensations of the intact breast as percepts mapped to other portions of the body. For example, the device can, in response to a pressure or other physical variable detected at the breast, generate a stimulus to cause a percept at the nape of the neck or the inner arm. This can be done in instances where it is difficult or impossible to provide stimulation to evoke a percept of the breast (e.g., where the remnant nerves are too damaged, where the breast requires re-operation, or some other circumstance) and/or to provide a redundancy. In such instances, an intervention and training period can precede the therapeutic intervention, in order to familiarize the patient with the new location of the stimulated percept.

### II. Example Systems

An implanted system as described herein may be configured in a variety of ways. For example, the controller, stimulators, sensors, of other electronic components of such a system may be configured in a variety of ways in order to provide the functionality described herein.

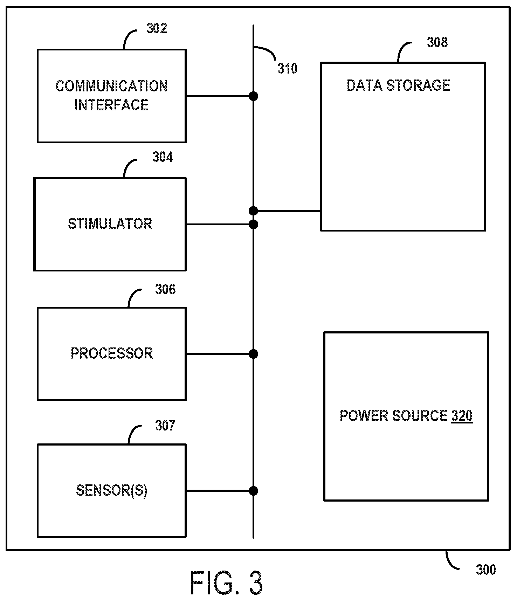

Figure 3 illustrates an example of such an implantable system 300, which may be used to implement the methods described herein. The example system 300 includes a communication interface 302, a stimulator 304, a processor 306, one or more sensors 307 (e.g., a flexible sensor that includes an array of capacitive or otherwise configured sensor elements), and data storage 308, all of which are communicatively linked together by a system bus 310. The system 300 also includes a power source 320 to power the other elements of the system 300. Such a power source 320 can include one or more chargeable or non-rechargeable batteries. In some examples, the power source 320 can include a coil or other means for receiving wireless power. Such received wireless power can be used to power the system 300 and/or to recharge one or more rechargeable batteries of the power source 320.

The communication interface 302 may function to allow the computing system 300 to communicate, using analog or digital modulation of electric, magnetic, electromagnetic, optical, or other signals, with other devices, access networks, and/or transport networks. For instance, communication interface 302 may include a chipset and antenna arranged for wireless communication with a radio access network or an access point. Also, communication interface 302 may take the form of or include a magnetometer or other sensor configured to permit detection of local magnetic fields such as those that may be generated from a control pendant or other device used to control the operation of the system 300, e.g., to activate or deactivate the system 300 or to control an operational mode of the system 300 in some other manner. Furthermore, communication interface 302 may comprise multiple physical communication interfaces (e.g., a WiFi interface, a BLUETOOTH^{®} interface, and a wide-area wireless interface).

In some embodiments, the communication interface 302 may function to allow the system 300 to communicate with other devices. For example, the communication interface 302 may function to transmit and/or receive an indication of mappings between detected sensor signals and an amplitude, frequency, polarity, waveform, identity of electrodes to activate, or other properties of an electrical stimulus to be provides to nerve(s) of a person so as to provide sensory function to the person. In examples wherein the power source 320 includes a coil or other means for receiving wireless power, the communication interface 302 may use such a coil or other means to effect wireless communication, as well, e.g., by engaging in backscatter communication.

The stimulator 304 of such a system 300 can include one or more pulse generators, voltage sources (e.g., boost converters, voltage multipliers), amplifiers, digital-to-analog-converters, current sources, electrical switches, or other elements to facilitate the generation of one or more stimulus waveforms. The stimulator 304 can also include a plurality of electrodes via which such stimulus waveforms can be delivered to nerves or to portions thereof (e.g., to branches of nerves, to individual fascicles or nerve fibers within the nerves). Such electrodes can be included in self-sizing cuffs, flat interface nerve electrode housings, or others housings to facilitate coupling the electrodes to or proximate to a nerve or portion thereof of interest. Additionally or alternatively, the stimulator 304 can deliver stimulus waveforms to nerves optically or by some other method. For example, the nerves can be made sensitive to one or more wavelengths of light (e.g., by being transfected with genes coding for one or more proteins that can transduce photons into transmembrane currents or some other biological stimulus), and the stimulator 304 can include lasers, light emitting diodes, or other elements configured to provide optical stimulus at the one or more wavelengths.

Processor 306 may comprise one or more general purpose processors - e.g., microprocessors - and/or one or more special purpose processors - e.g., digital signal processors (DSPs), graphics processing units (GPUs), floating point units (FPUs), network processors, tensor processing units (TPUs), or application-specific integrated circuits (ASICs). Data storage 308 may include one or more volatile and/or non-volatile storage components, such as magnetic, optical, flash, or organic storage, and may be integrated in whole or in part with processor 306. Data storage 308 may include removable and/or non-removable components.

Processor 306 may be capable of executing program instructions (e.g., compiled or non-compiled program logic and/or machine code) stored in data storage 308 to carry out the various functions described herein. Therefore, data storage 308 may include a non-transitory computer-readable medium, having stored thereon program instructions that, upon execution by system 300, cause system 300 to carry out any of the methods, processes, or functions disclosed in this specification and/or the accompanying drawings.

The sensor(s) 307 can be configured in a variety of ways to facilitate detection of pressure, strain, vibration, temperature, or other physical variables related to percepts of interest. The sensor 307 can include one or more strain sensors, pressure sensors, temperature sensors, accelerometers, or other elements configured to detect a physical variable related to a pressure, strain, vibration, temperature, or other physical variable related to a percept of interest. The sensor 307 can be a flexible sensor, e.g., having a compliance approximating the compliance of skin, breast tissue, or some other tissue in or near which the sensor 307 is to be surgically implanted. Such a flexible sensor can include a number of non-flexible elements (e.g., discrete transistors, integrated circuits, sensor elements) embedded in a flexible substrate such that the sensor is, overall, flexible. Additionally or alternatively, such a flexible sensor can include flexible transistors, flexible capacitors (e.g., an array of flexible capacitive sensor elements that can be used to detect pressure, strain, vibration, or other physical variables), or other flexible electronic elements. Such elements can be formed from or otherwise integrated into a flexible substrate material, e.g., a flexible conductive or semiconductive material. The sensor can include tabs or other features to facilitate surgically fixing the sensor to anatomy of interest (e.g., to an internal surface of skin). A flexible or otherwise configured sensor may also include a plurality of holes or other features formed therein to facilitate through-growth of tissue, securing the sensor to adjacent tissues. Such through-growth may allow resorbable sutures, clips, or other resorbable materials to be used to initially fix the sensor to adjacent tissues and later to be resorbed after the through-growth has occurred. This can have the benefit of avoiding the potentially negative tactile or other effects of sutures, clips, or other securing elements long after implantation of the sensor has occurred.

### III. Example Methods

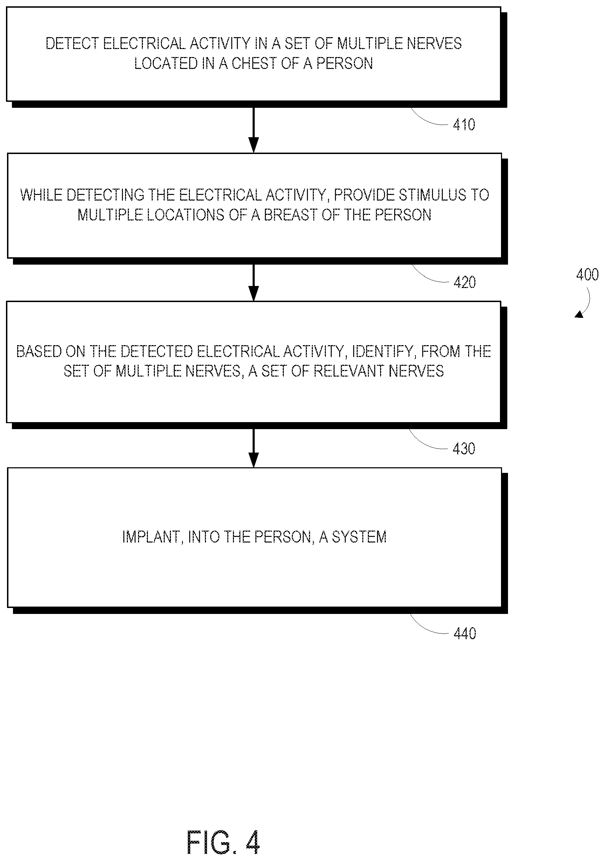

Figure 4 is a flowchart of an exemplary method 400 for identifying which nerves of a person (e.g., roots and/or branches of intercostal nerves) conduct sensory information from the breast of the person, so as to later implant a neuroprosthetic device to restore that sensory information following reconstructive surgery or other intervention that can disrupt that sensation.

The method 400 includes detecting electrical activity in a set of multiple nerves located in a chest of a person (410). This can include inserting and/or attaching electrodes to one or more of the intercostal nerves of the person (e.g., the third, fourth, and/or fifth intercostal nerves) and/or to one or more branches thereof (e.g., one or more branches of an intercostal nerve). Such electrodes can include contact electrodes, penetrating electrodes, one or more arrays of electrodes, or some other type or combination of types of electrodes. The detected electrical activity can be local field potentials that are indicative of the activity of populations of nerve fibers within a nerve.

The method 400 additionally includes, while detecting the electrical activity, providing stimulus to multiple locations of a breast of the person (420). This can include a surgeon or other healthcare provider manually providing the stimulus. Additionally or alternatively, the stimulus can be provided by an electrical stimulator, a mechanical vibrator or other mechanical actuator, a fan, or some other means to provide the stimulus. The stimulus can be provided to a specified set of locations, e.g., to a set of locations specified pre-operatively. The set of locations can be determined based on feedback from the person regarding the types and locations of sexual or other stimulation that are most valuable

The method 400 additionally includes, based on the detected electrical activity, identifying, from the set of multiple nerves, a set of relevant nerves (430). This can be performed by a surgeon or other healthcare provider, e.g., based on correspondences in timing of the stimulus and a visually or acoustically-presented indication of the detected electrical activity in one or more of the multiple nerves. Additionally or alternatively, a computer can be used to identify the set of relevant nerves based on recordings of the detected electrical activity and one or more signals related to the provision of the provided stimulus, e.g., a signal related to the pressing of a button by a surgeon when the surgeon is providing stimulus to the breast.

The method 400 further includes implanting, into the person, a system (440). The system includes: (i) a sensor, wherein the sensor is configured to detect at least one of a pressure, a strain, a vibration, or a temperature; (ii) a stimulator, wherein the stimulator is configured to stimulate one or more (e.g., at least two) nerves, and wherein the stimulator comprises a plurality of electrodes; and (iii) a controller that is operably coupled to the sensor and the stimulator and that is configured to: (a) operate the sensor to detect at least one of a pressure, a strain, a vibration, or a temperature; and (b) based on the detected at least one of a pressure, a strain, a vibration, or a temperature, operate the stimulator to provide a stimulus to at least one of the at least two nerves. Implanting the system includes coupling each electrode of the plurality of electrodes to a respective nerve of the set of relevant nerves. Where more nerves are present in the set of relevant nerves than can be coupled to respective electrodes of the system (e.g., due to the system having fewer electrodes and/or electrode housings than there are nerves in the set of relevant nerves), a subset of the set of relevant nerves can be coupled to respective electrodes. Such a subset can be selected based on the strength of response of the nerves to the stimulus, based on the user's preferences, or based on some other consideration.

The method 400 can include additional elements or features.

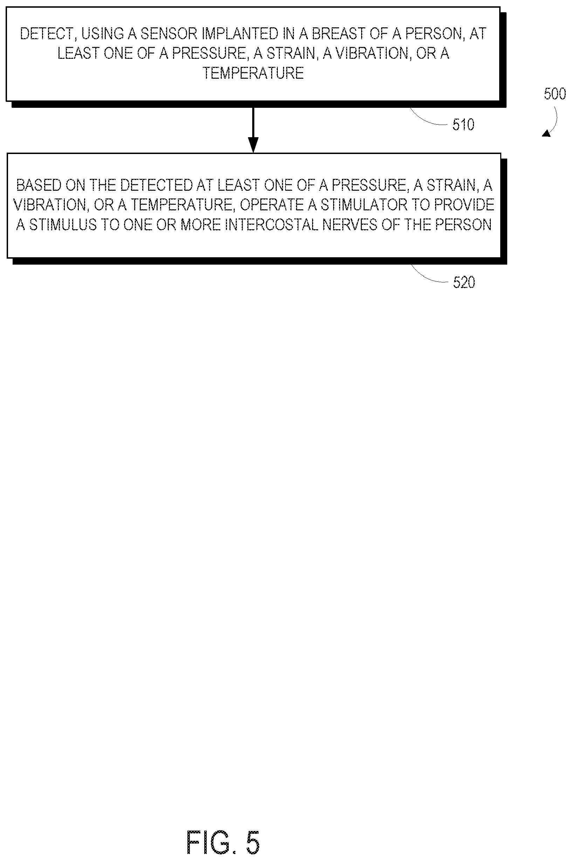

Figure 5 is a flowchart of an exemplary method 500 for restoring breast sensation to a person. The method 500 includes detecting, using a sensor implanted in a breast of a person, at least one of a pressure, a strain, a vibration, or a temperature (510) and, based on the detected at least one of a pressure, a strain, a vibration, or a temperature, operating a stimulator to provide a stimulus to one or more intercostal nerves of the person (520). The method 500 can include additional elements or features.

### IV. Conclusion

The above detailed description describes various features and functions of the disclosed systems, devices, and methods with reference to the accompanying figures. In the figures, similar symbols typically identify similar components, unless context indicates otherwise. The illustrative embodiments described in the detailed description, figures, and claims are not meant to be limiting. Other embodiments can be utilized, and other changes can be made, without departing from the scope of the present invention which is defined by the following claims.

## Claims

1. A system (100) comprising:
a flexible sensor (120, 210a, 210b, 210c), wherein the sensor (120, 210a, 210b, 210c) is configured for subcutaneous and/or subdermal placement and comprises a plurality of apertures to permit through-growth of tissue, and wherein the sensor (120, 210a, 210b, 210c) is configured to detect at least one of a pressure, a strain, a vibration, or a temperature;
a stimulator, wherein the stimulator is configured to stimulate at least one nerve; and
a controller (110), wherein the controller (110) is operably coupled to the sensor (120, 210a, 210b, 210c) and the stimulator, and wherein the controller (110) is configured to:
operate the sensor (120, 210a, 210b, 210c) to detect at least one of a pressure, a strain, a vibration, or a temperature; and
based on the detected at least one of a pressure, a strain, a vibration, or a temperature, operate the stimulator to provide a stimulus to the at least one nerve.

2. The system (100) of claim 1, wherein the sensor (120, 210a, 210b, 210c) is configured to be surgically secured to an internal surface of skin.

3. The system (100) of claim 1, wherein the sensor (120, 210a, 210b, 210c) has a compliance that is within 30% of a compliance of human breast tissue or within 30% of a compliance of human skin tissue.

4. The system (100) of any of claims 1-4, wherein the stimulator comprises:
a first housing (130a) configured to be mechanically coupled to a first nerve; and
a first electrode coupled to the first housing (130a) such that, when the first housing (130a) is coupled to the nerve, an electrical stimulus can be provided to the first nerve via the first electrode.

5. The system (100) of claim 4, wherein the stimulator comprises:
a second housing (130b) configured to be mechanically coupled to a second nerve; and
a second electrode coupled to the second housing (130b) such that, when the second housing (130b) is coupled to the second nerve, an electrical stimulus can be provided to the second nerve via the second electrode.

6. The system (100) of claim 5, wherein the controller (110) is configured for: (i) during a first period of time, detecting a first sensor signal from a first region of the sensor (120, 210a, 210b, 210c), and (ii) during a second period of time, detecting a second sensor signal from a second region of the sensor (120, 210a, 210b, 210c), wherein the second region differs from the first region, wherein the stimulator is configured for (i) during the first period of time, providing, to the first nerve via the first electrode, a first stimulus based on the first sensor signal, and (ii) during the second period of time, providing, to the second nerve via the second electrode, a second stimulus based on the second sensor signal, and wherein the second nerve differs from the first nerve.

7. The system (100) of claim 5, wherein the controller (110) is configured for, during a first period of time, detecting a first sensor signal from a first region of the sensor (120, 210a, 210b, 210c), and wherein the stimulator is configured for, during the first period of time: (i) providing, to the first nerve via the first electrode, a first stimulus based on the first sensor signal, and (ii) providing, to the second nerve via the second electrode, a second stimulus based on the first sensor signal, wherein the second nerve differs from the first nerve.

8. The system (100) of claim 1, wherein the sensor (120, 210a, 210b, 210c) comprises a plurality of flexible capacitive sensor elements.

9. The system (100) of claim 1, wherein the controller (110) is additionally configured to:
operate in a first mode, wherein operating in the first mode comprises:
operating the sensor (120, 210a, 210b, 210c) to detect a first at least one of a pressure, a strain, a temperature, or a vibration;
determining, based on the detected first at least one of a pressure, a strain, a temperature, or a vibration, a first stimulus waveform according to a first mapping; and
operating the stimulator to provide the determined first stimulus waveform; receive a mode change instruction; and
responsive to receiving the mode change instruction, operate in a second mode, wherein operating in the second mode comprises:
operating the sensor (120, 210a, 210b, 210c) to detect a second at least one of a pressure, a strain, a temperature, or a vibration;
determining, based on the detected second at least one of a pressure, a strain, a temperature, or a vibration, a second stimulus waveform according to a second mapping, wherein the second mapping differs from the first mapping; and
operating the stimulator to provide the determined second stimulus waveform.

10. The system (100) of claim 9, further comprising a wireless receiver, wherein receiving a mode change instruction comprises operating the wireless receiver to detect a wireless indication of the mode change instruction.

11. The system (100) of any preceding claim, further comprising:
a breast implant (230a, 230b, 230c);
wherein the sensor (120, 210a, 210b, 210c) is disposed on or within the breast implant (230a, 230b, 230c) proximate to a surface of the breast implant (230a, 230b, 230c), wherein the breast implant (230a, 230b, 230c) is configured for subcutaneous placement.

12. The system (100) of claim 11, wherein the surface of the breast implant (230a, 230b, 230c) is configured to be surgically secured to an internal surface of skin.

## Patentansprüche

1. System (100), umfassend:
einen flexiblen Sensor (120, 210a, 210b, 210c), wobei der Sensor (120, 210a, 210b, 210c) für die subkutane und/oder subdermale Platzierung konfiguriert ist und eine Vielzahl von Öffnungen aufweist, um das Hindurchwachsen von Gewebe zu ermöglichen, und wobei der Sensor (120, 210a, 210b, 210c) konfiguriert ist, um mindestens eines von einem Druck, einer Dehnung, einer Vibration oder einer Temperatur zu erfassen;
einen Stimulator, wobei der Stimulator konfiguriert ist, um mindestens einen Nerv zu stimulieren; und
eine Steuerung (110), wobei die Steuerung (110) funktionsfähig mit dem Sensor (120, 210a, 210b, 210c) und dem Stimulator gekoppelt ist und wobei die Steuerung (110) konfiguriert ist zum:
Betätigen des Sensors (120, 210a, 210b, 210c), um mindestens eines von einem Druck, einer Dehnung, einer Vibration oder einer Temperatur zu erfassen; und
Betätigen des Stimulators auf der Grundlage des erfassten mindestens einen von einem Druck, einer Dehnung, einer Vibration oder einer Temperatur, , um einen Stimulus an den mindestens einen Nerv bereitzustellen.

2. System (100) nach Anspruch 1, wobei der Sensor (120, 210a, 210b, 210c) konfiguriert ist, um chirurgisch an einer inneren Oberfläche der Haut befestigt zu werden.

3. System (100) nach Anspruch 1, wobei der Sensor (120, 210a, 210b, 210c) eine Nachgiebigkeit aufweist, die innerhalb von 30 % der Nachgiebigkeit von menschlichem Brustgewebe oder innerhalb von 30 % der Nachgiebigkeit von menschlichem Hautgewebe liegt.

4. System (100) nach einem der Ansprüche 1 bis 4, wobei der Stimulator umfasst:
ein erstes Gehäuse (130a), das konfiguriert ist, um mechanisch mit einem ersten Nerv gekoppelt zu werden; und
eine erste Elektrode, die mit dem ersten Gehäuse (130a) so gekoppelt ist, dass, wenn das erste Gehäuse (130a) mit dem Nerv gekoppelt ist, ein elektrischer Stimulus über die erste Elektrode an den ersten Nerv bereitgestellt werden kann.

5. System (100) nach Anspruch 4, wobei der Stimulator umfasst:
ein zweites Gehäuse (130b), das konfiguriert ist, um mechanisch mit einem zweiten Nerv gekoppelt zu werden; und
eine zweite Elektrode, die mit dem zweiten Gehäuse (130b) so gekoppelt ist, dass, wenn das zweite Gehäuse (130b) mit dem zweiten Nerv gekoppelt ist, ein elektrischer Stimulus über die zweite Elektrode an den zweiten Nerv bereitgestellt werden kann.

6. System (100) nach Anspruch 5, wobei die Steuerung (110) konfiguriert ist zum:
(i) während eines ersten Zeitraums, Erfassen eines ersten Sensorsignals aus einem ersten Bereich des Sensors (120, 210a, 210b, 210c), und (ii) während eines zweiten Zeitraums, Erfassen eines zweiten Sensorsignals aus einem zweiten Bereich des Sensors (120, 210a, 210b, 210c), wobei der zweite Bereich sich von dem ersten Bereich unterscheidet, wobei der Stimulator konfiguriert ist zum
(i) während des ersten Zeitraums, Bereitstellen eines ersten Stimulus, der auf dem ersten Sensorsignal basiert, an den ersten Nerv über die erste Elektrode, und (ii) während des zweiten Zeitraums, Bereitstellen eines zweiten Stimulus, der auf dem zweiten Sensorsignal basiert, an den zweiten Nerv über die zweite Elektrode, wobei sich der zweite Nerv von dem ersten Nerv unterscheidet.

7. System (100) nach Anspruch 5, wobei die Steuerung (110) konfiguriert ist zum, während eines ersten Zeitraums, Erfassen eines ersten Sensorsignals von einem ersten Bereich des Sensors (120, 210a, 210b, 210c), und wobei der Stimulator konfiguriert ist zum,
während des ersten Zeitraums: i) Bereitstellen eines ersten Stimulus, der auf dem ersten Sensorsignal basiert, an den ersten Nerv über die erste Elektrode, und ii) Bereitstellen eines zweiten Stimulus, der auf dem ersten Sensorsignal basiert, an den zweiten Nerv über die zweite Elektrode, wobei der zweite Nerv sich von dem ersten Nerv unterscheidet.

8. System (100) nach Anspruch 1, wobei der Sensor (120, 210a, 210b, 210c) eine Vielzahl flexibler kapazitiver Sensorelemente umfasst.

9. System (100) nach Anspruch 1, wobei die Steuerung (110) zusätzlich konfiguriert ist zum:
Betreiben in einem ersten Modus, wobei das Betreiben im ersten Modus umfasst:
Betreiben des Sensors (120, 210a, 210b, 210c), um mindestens eines von einem Druck, einer Dehnung, einer Temperatur oder einer Vibration zu erfassen;
Bestimmen einer ersten Stimulus-Wellenform gemäß einer ersten Zuordnung auf der Grundlage des erfassten ersten mindestens einen von einem Druck, einer Dehnung, einer Temperatur oder einer Vibration;
und
Betreiben des Stimulators, um die bestimmte erste Stimulus-Wellenform bereitzustellen;
Empfangen einer Modusänderungsanweisung; und
als Reaktion auf das Empfangen der Modusänderungsanweisung, Betreiben in einem zweiten Modus,
wobei das Betreiben in dem zweiten Modus umfasst:
Betreiben des Sensors (120, 210a, 210b, 210c), um ein zweites mindestens eines von einem Druck, einer Dehnung, einer Temperatur oder einer Vibration zu erfassen;
Bestimmen einer zweiten Stimulus-Wellenform gemäß einer zweiten Zuordnung auf der Grundlage des erfassten zweiten mindestens einen von einem Druck, einer Dehnung, einer Temperatur oder einer Vibration, wobei die zweite Zuordnung sich von der ersten Zuordnung unterscheidet; und
Betreiben des Stimulators, um die bestimmte zweite Stimulus-Wellenform bereitzustellen.

10. System (100) nach Anspruch 9, ferner umfassend einen drahtlosen Empfänger, wobei das Empfangen einer Modusänderungsanweisung das Betreiben des drahtlosen Empfängers umfasst, um eine drahtlose Angabe der Modusänderungsanweisung zu erfassen.

11. System (100) nach einem der vorstehenden Ansprüche, ferner umfassend:
ein Brustimplantat (230a, 230b, 230c);
wobei der Sensor (120, 210a, 210b, 210c) auf oder in dem Brustimplantat (230a, 230b, 230c) in der Nähe einer Oberfläche des Brustimplantats (230a, 230b, 230c) angeordnet ist, wobei das Brustimplantat (230a, 230b, 230c) für die subkutane Platzierung konfiguriert ist.

12. System (100) nach Anspruch 11, wobei die Oberfläche des Brustimplantats (230a, 230b, 230c) konfiguriert ist, um chirurgisch an einer inneren Oberfläche der Haut befestigt zu werden.

## Revendications

1. Système (100) comprenant :
un capteur flexible (120, 210a, 210b, 210c), dans lequel le capteur (120, 210a, 210b, 210c) est configuré pour un placement sous-cutané et/ou sous-dermique et comprend une pluralité d'ouvertures pour permettre la traversée de tissu, et dans lequel le capteur (120, 210a, 210b, 210c) est configuré pour détecter au moins un élément parmi une pression, une contrainte, une vibration ou une température ;
un stimulateur, dans lequel le stimulateur est configuré pour stimuler au moins un nerf ; et
un dispositif de commande (110), dans lequel le dispositif de commande (110) est couplé opérationnellement au capteur (120, 210a, 210b, 210c), et au stimulateur, et dans lequel le dispositif de commande (110) est configuré pour :
actionner le capteur (120, 210a, 210b, 210c) afin de détecter au moins un élément parmi une pression, une contrainte, une vibration ou une température ; et
sur la base de l'au moins un élément détecté parmi une pression, une contrainte, une vibration ou une température, actionner le stimulateur afin de fournir un stimulus à le au moins un nerf.

2. Système (100) selon la revendication 1, dans lequel le capteur (120, 210a, 210b, 210c) est configuré pour être chirurgicalement fixé à une surface interne de la peau.

3. Système (100) selon la revendication 1, dans lequel le capteur (120, 210a, 210b, 210c) présente une conformité qui est d'environ 30 % maximum conforme au tissu d'un sein humain ou d'environ 30 % maximum conforme au tissu d'une peau humaine.

4. Système (100) selon l'une quelconque des revendications 1 à 4, dans lequel le stimulateur comprend :
un premier logement (130a) configuré pour être mécaniquement couplé à un premier nerf ; et
une première électrode couplée au premier logement (130a) de sorte que, lorsque le premier logement (130a) est couplé au nerf, un stimulus électrique peut être fourni au premier nerf via la première électrode.

5. Système (100) selon la revendication 4, dans lequel le stimulateur comprend :
un deuxième logement (130b) configuré pour être mécaniquement couplé à un deuxième nerf ; et
une deuxième électrode couplée au deuxième logement (130b) de sorte que, lorsque le deuxième logement (130b) est couplé au deuxième nerf, un stimulus électrique peut être fourni au deuxième nerf via la deuxième électrode.

6. Système (100) selon la revendication 5, dans lequel le dispositif de commande (110) est configuré pour :
(i) pendant un premier laps de temps, détecter un premier signal de capteur depuis une première région du capteur (120, 210a, 210b, 210c), et (ii) pendant un deuxième laps de temps, détecter un deuxième signal de capteur depuis une deuxième région du capteur (120, 210a, 210b, 210c), dans lequel la deuxième région diffère de la première région, dans lequel le stimulateur est configuré pour :
(i) pendant le premier laps de temps, fournir au premier nerf, via la première électrode, un premier stimulus basé sur le premier signal de capteur, et (ii) pendant le deuxième laps de temps, fournir au deuxième nerf via la deuxième électrode, un deuxième stimulus basé sur le deuxième signal de capteur, et dans lequel le deuxième nerf diffère du premier nerf.

7. Système (100) selon la revendication 5, dans lequel le dispositif de commande (110) est configuré pour :
pendant un premier laps de temps, détecter un premier signal de capteur depuis une première région du capteur (120, 210a, 210b, 210c), et dans lequel le stimulateur est configuré pour, pendant le premier laps de temps, (i) fournir au premier nerf, via la première électrode, un premier stimulus basé sur le premier signal de capteur, et (ii) fournir au deuxième nerf, via la deuxième électrode, un deuxième stimulus basé sur le premier signal de capteur, dans lequel le deuxième nerf diffère du premier nerf.

8. Système (100) selon la revendication 1, dans lequel le capteur (120, 210a, 210b, 210c) comprend une pluralité d'éléments de capteur capacitif flexible.

9. Système (100) selon la revendication 1, dans lequel le dispositif de commande (110) est en outre configuré pour :
fonctionner dans un premier mode, dans lequel le fonctionnement dans le premier mode comprend :
l'actionnement du capteur (120, 210a, 210b, 210c), afin de détecter un premier au moins un élément parmi une pression, une contrainte, une température ou une vibration ;
la détermination, sur la base du premier élément détecté, à savoir au moins un élément parmi une pression, une contrainte, une température ou une vibration, d'une première forme d'onde de stimulus selon une première cartographie ; et
l'actionnement du stimulateur pour fournir la première forme d'onde de stimulus déterminée ;
recevoir une instruction de changement de mode ; et
en réponse à la réception de l'instruction de changement de mode, l'actionnement dans un deuxième mode, dans lequel l'actionnement dans le deuxième mode comprend :
l'actionnement du capteur (120, 210a, 210b, 210c), pour détecter un deuxième au moins un élément parmi une pression, une contrainte, une température ou une vibration ;
la détermination, sur la base du deuxième élément détecté, à savoir au moins un élément parmi une pression, une contrainte, une température ou une vibration, d'une deuxième forme d'onde de vibration selon une deuxième cartographie, dans lequel la deuxième cartographie diffère de la première cartographie ; et
l'actionnement du stimulateur pour fournir la deuxième forme d'onde de stimulus déterminée.

10. Système (100) selon la revendication 9, comprenant en outre un récepteur sans fil, dans lequel la réception d'une instruction de changement de mode comprend l'actionnement du récepteur sans fil pour détecter une indication sans fil de l'instruction de changement de mode.

11. Système (100) selon l'une quelconque des revendications précédentes, comprenant en outre :
un implant mammaire (230a, 230b, 230c),
dans lequel le capteur (120, 210a, 210b, 210c) est disposé sur ou dans l'implant mammaire (230a, 230b, 230c) près d'une surface de l'implant mammaire (230a, 230b, 230c), dans lequel l'implant mammaire (230a, 230b, 230c) est configuré pour un placement sous-cutané.

12. Système (100) selon la revendication 11, dans lequel la surface de l'implant mammaire (230a, 230b, 230c) est configurée pour être chirurgicalement fixée à une surface interne de la peau.
